(19) Europäisches Patentamt
European Patent Office
Office européen des brevets

(11) Publication number: 0 154 185 B1

# (12) EUROPEAN PATENT SPECIFICATION

(45) Date of publication of patent specification: 05.10.88

(51) Int. Cl.$^4$: C 07 C 172/00, C 07 D 317/26, A 61 K 31/59

(21) Application number: 85101254.2

(22) Date of filing: 06.02.85

## (54) Calciferol derivatives.

(30) Priority: 08.02.84 US 578160
17.12.84 US 682125

(43) Date of publication of application:
11.09.85 Bulletin 85/37

(45) Publication of the grant of the patent:
05.10.88 Bulletin 88/40

(84) Designated Contracting States:
AT BE CH DE FR GB IT LI LU NL SE

(56) References cited:
EP-A-0 086 350
EP-A-0 086 476
EP-A-0 115 314
WO-A-84/00107

(73) Proprietor: F. HOFFMANN-LA ROCHE & CO.
Aktiengesellschaft
CH-4002 Basel (CH)

(72) Inventor: Baggiolini, Enrico Giuseppe
30 Evergreen Drive
North Caldwell, N.J. (US)
Inventor: Batcho, Andrew David
19 White Oak Drive
North Caldwell, N.J. (US)
Inventor: Truitt, Gary Arthur
38 Stewart Street
Passaic, N.J. (US)
Inventor: Uskokovic, Milan Radoje
253 Highland Avenue
Upper Montclair, N.J. (US)
Inventor: Wovkulich, Peter Michael
124 Rhoda Avenue
Nutley, N.J. (US)

(74) Representative: Lederer, Franz, Dr. et al
Van der Werth, Lederer & Riederer
Patentanwälte Lucile-Grahn-Strasse 22
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 154 185 B1

Courier Press, Leamington Spa, England.

**Description**

The present invention relates to a process for the preparation of calciferol derivatives of the formula

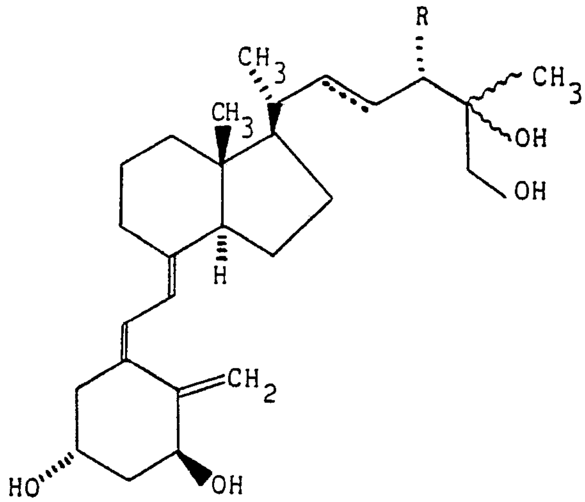

I

wherein R is hydrogen and the dotted line in the side chain is an optional additional C—C-bond, or R is methyl and the dotted line in the side chain is an additional C—C-bond, in the 25R or 25S epimeric form or as mixtures thereof.

The invention also relates to novel compounds of the formula

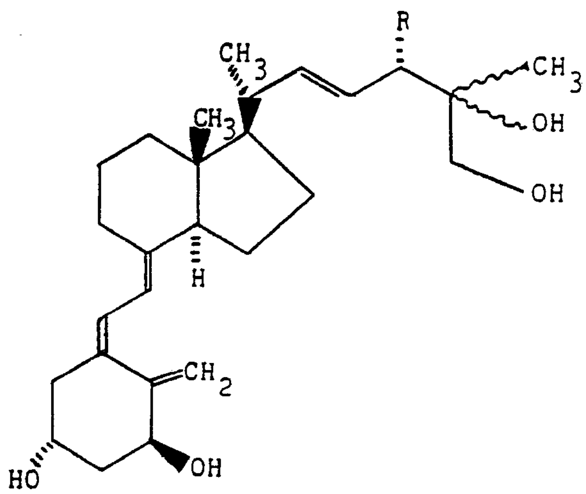

I-A

wherein R is hydrogen or methyl, in the 25R or 25S epimeric form or as mixtures thereof, to pharmaceutical compositions containing a compound of formula I or IA, and to novel intermediates occurring in the preparation of these compounds.

As used herein, the term "lower alkyl" denotes a straught or branched-chain saturated hydrocarbon group preferably containing from 1 to 6 C-atoms, e.g. methyl, ethyl, propyl, isopropyl, butyl, t-butyl, pentyl or hexyl. The term "$C_{3-6}$ alkylene" denotes a straight or branched-chain organic radical derived from an unsaturated aliphatic hydrocarbon group, e.g. propylene or butylene. The term "aryl" denotes phenyl or phenyl bearing one or more substituents selected from the group consisting of $C_{1-6}$ alkyl and $C_{1-6}$ alkoxy.

The process for preparing the compounds of formula I comprises reacting the corresponding specific epimer of the formula

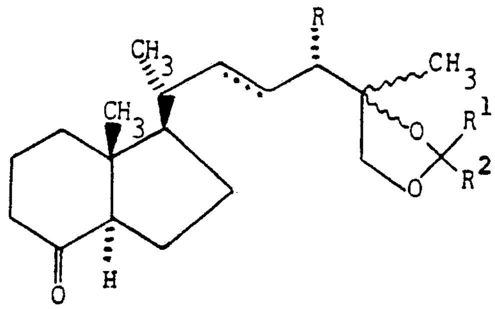

II

wherein R and the dotted line in the side chain have the above significance and $R^1$ and $R^2$ are hydrogen, lower alkyl or aryl, or taken together are $C_{3-6}$-alkylene, with a compound of the formula

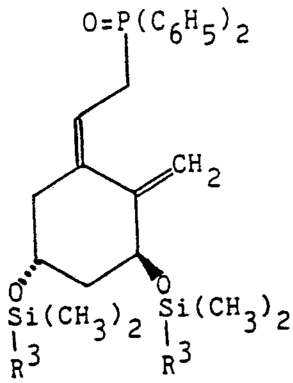

III

wherein $R^3$ is lower alkyl or aryl, and treating the obtained compound of the formula

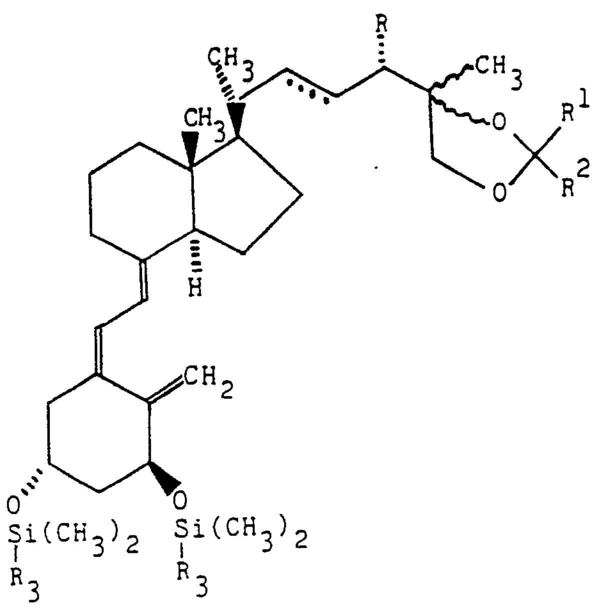

IV

wherein R, $R^1$, $R^2$, $R^3$ and the dotted line in the side chain have the above significance with a deprotecting agent.

The reaction of a ketone II with a phosphine oxide III can be carried out in the presence of a base in a conventional ether solvent, e.g. tetrahydrofuran (THF) or dioxane, under an inert atmosphere, e.g. of argon, at a temperature in the range of from about −80 to −50°C. Exemplary of suitable bases are alkyl lithium compounds, e.g. n-butyl-lithium, and alkali metal dialkyl or disilyl amides. The compound of formula IV can be purified, e.g. by elution chromatography on silica gel.

The compound of formula IV can be converted to the corresponding compound of formula I by treatment with a deprotecting agent, such as an alkanol, e.g. ethanol, or water, in the presence of an acid. While any mineral acid or lower alkanoic or sulfonic acid may be used it is preferred to use a cationic ion

exchange resin as a suspension in a lower alkanol. The corresponding compound of the formula I can be isolated by removal of the solid cationic exchange resin by filtration and evaporation of the volatiles under reduced pressure.

EP—A—86350 and EP—A—86476 relate to cholecalciferol derivatives which in contradistinction to the calciferol derivatives of formula I—A of the instant invention, are saturated in the 22,23-position. The compounds described in WO—A—84/00107 contain a glycosidic residue, of which the compounds of formula I—A are devoid. The cholecalciferol derivatives described in EP—A—86350 are obtained from cholesterol derivatives. In the process of the instant invention the calciferol derivatives of formula I are obtained from indenone derivatives of formula II and the phosphine oxides of formula III.

The starting ketones of formula II, wherein R is hydrogen, can be prepared as set forth in the following reaction scheme 1 wherein $R^1$, $R^2$ and $R^3$ are as above.

<u>Reaction scheme 1</u>

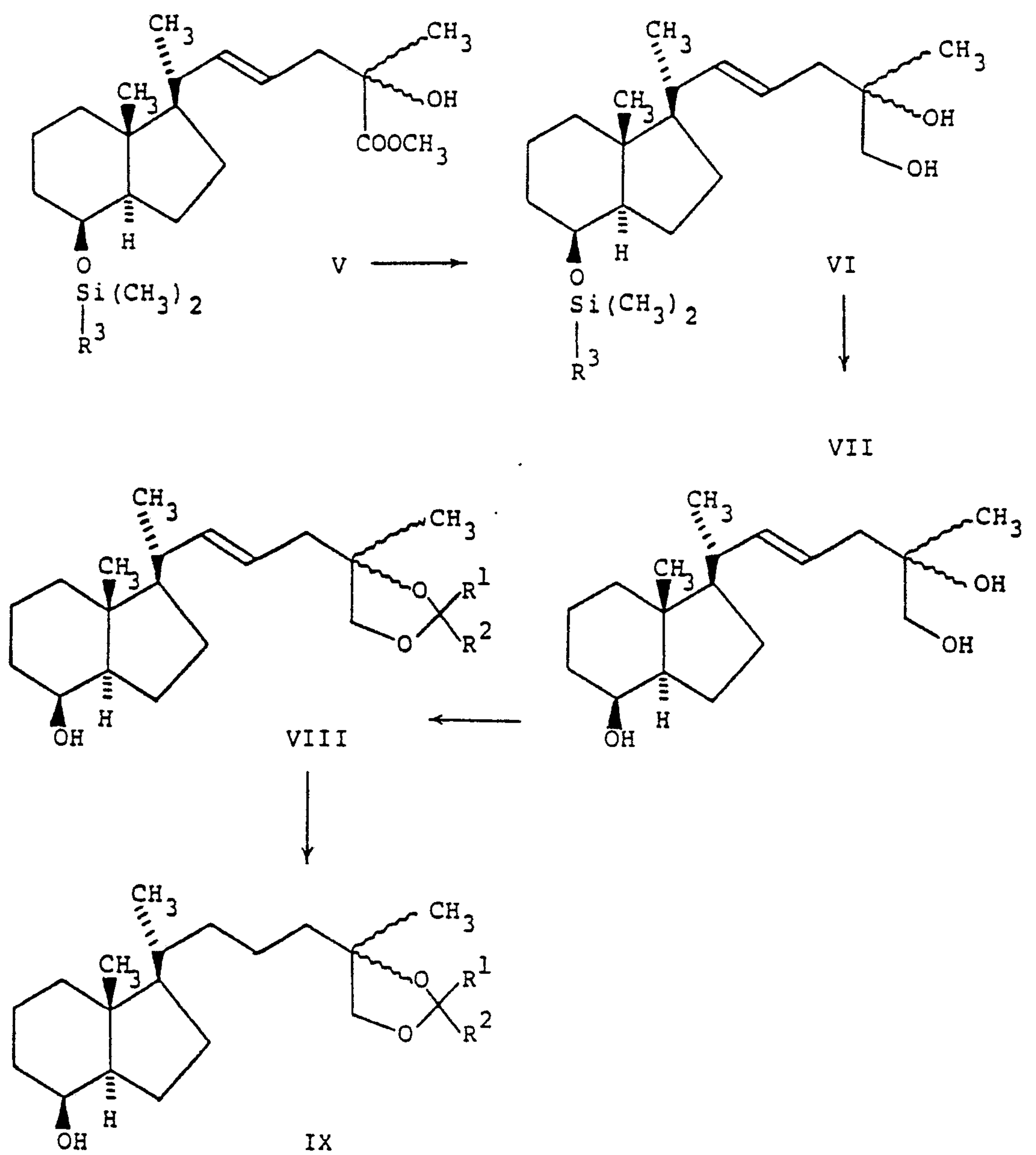

V VI VII VIII IX

A compound of formula V can be reduced with lithium aluminum hydride ($LiAlH_4$) in an anhydrous conventional ether solvent, e.g. THF, at 0°C, to give the corresponding diol VI. Conversion of the latter to the corresponding triol VII can be carried out by removal of the protecting group. e.g. the t-butyl-dimethylsilyl group, with a 48% aqueous hydrogen fluoride solution in a mixture of acetonitrile and THF. Protection of the vicinal diol of the side chain in a compound of formula VII by conversion to the acetonide VIII can be accomplished by treatment, e.g. with 2,2-dimethoxypropane and catalyzed, e.g. with p-toluenesulfonic

acid. Conversion of the resulting compound of formula VIII to the dihydro analog of formula IX can be achieved by hydrogenation over a 10% palladium on carbon catalyst at atmospheric pressure in a solvent, e.g. ethyl acetate. Finally, the corresponding ketone II can be obtained by oxidation of a compound of formula VIII or IX, e.g. with 2,2'-bipyridinium chlorochromate in a solvent, e.g. methylene chloride, and in the presence of anhydrous sodium acetate.

The starting ketones of formula II, wherein R is methyl, can be prepared as set forth in the following reaction scheme 2 wherein $R^1$ and $R^2$ are as above and Ts is the p-toluenesulfonyl group.

Reaction scheme 2

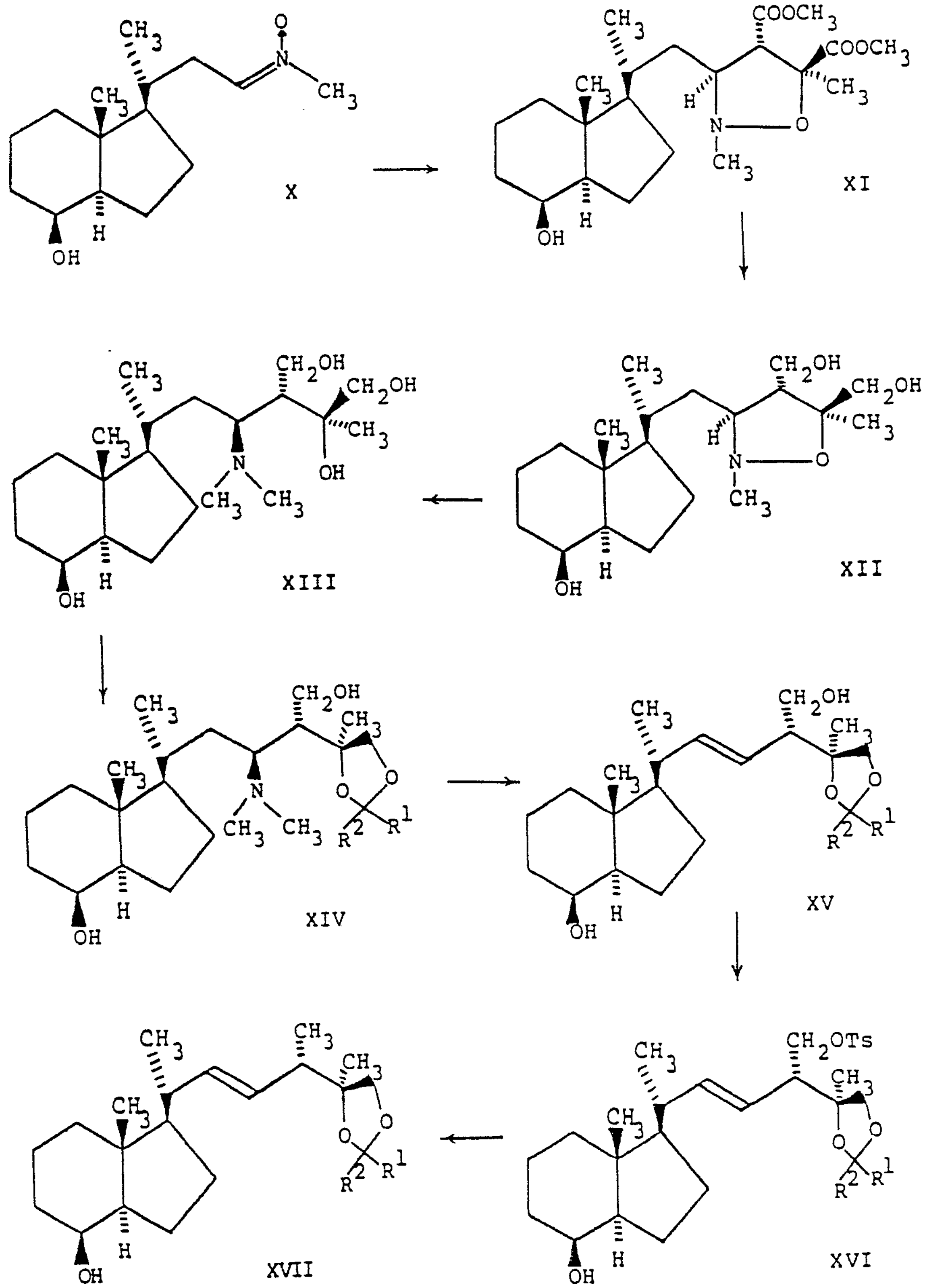

X XI XIII XII XIV XV XVII XVI

The compound of formula X can be heated, e.g. with methyl mesaconate in an anhydrous conventional aromatic solvent, e.g. xylene, at 140°C, to give the isoxazolidine diester XI. Conversion of the latter to the corresponding diol XII can be carried out by reduction with $LiAlH_4$ in an anhydrous conventional ether solvent, e.g. THF, at an ice bath temperature. Opening of the isoxazolidine ring to produce the compound of the formula XIII can be carried out by N-methylation, e.g. with methyliodide in toluene at 60°C, followed by reduction, e.g. with zinc dust in acetic acid solution at room temperature. Alternatively, this reduction can be performed with $LiAlH_4$ in THF. Protection of the vicinal diol of the side chain of the compound of formula XIII by conversion to the acetonide XIV can be accomplished as described above for the compound of formula VII. Conversion of the compound of formula XIV to that of formula XV can be carried out by methylation with methyliodide in dry toluene in the presence of potassium carbonate at 65°C, followed by elimination effected e.g. with potassium t-butylate at 55°C and finally at 100°C. The compound of formula XV is then converted to the tosylate XVI, e.g. with p-toluenesulfonyl chloride in methylene chloride in the presence of triethylamine. Reduction of the compound of formula XVI to the compound of formula XVII can be carried out with $LiAlH_4$ in THF at the reflux temperature. Finally, the ketone II can be obtained by oxidation of the compound of formula XVII, e.g. as described above for the compound of formula VIII or IX.

The compounds of formula I have anti-proliferative and differentiation-inducing activity and are therefore useful as agents for the treatment of diffuse tumor, such as leukemias, e.g. monocytic and promyelocytic leukemias, as well as solid tumors, e.g. lung carcinomas, melanoma, colorectal carcinoma, and breast tumors, in warm-blooded animals. The anti-proliferative and differentiation-inducing activity of the compounds of formula I can be demonstrated utilizing known procedures, e.g. as described in Progress in Cancer Research and Therapy, Vol. 23: Maturation Factors and Cancer, Ed. M.A.S. Moore, Raven Press, N.Y. 1982; Nature 270 (1977) 347-9 and Blood 54 (1979) 429-39.

The results obtained with the following compounds of formula I are set out in the Tables 1—4:

1α,25(S),26-trihydroxycholecalciferol[1α,25S,26-$(OH)_3D_3$]

1α,25(R),26-trihydroxycholecalciferol[1α,25R,26-$(OH)_3D_3$]

1α,25(S),26-trihydroxy-$\Delta^{22}$-cholecalciferol-[1α,25S,26—$(OH)_3$—$\Delta^{22}$—$D_3$]

1α,25(S),26-trihydroxyergocalciferol[1α,25S,26-$(OH)_3D_3$].

TABLE 1

Anti-proliferative and differentiation-inducing effects of 1α,25S,26—$(OH)_3D_3$; 1α,25R,26—$(OH)_3D_3$; 1α,25S,26—$(OH)_3$—$\Delta^{22}$—$D_3$ and 1α,25S,26—$(OH)_3$—$D_2$ at doses of 1 to 10 × $10^{-9}$ molar on HL—60 cells, in vitro.

| Compound and[a] concentration (× $10^{-9}$ molar) | | Proliferation[b] | | Differentiation | |
|---|---|---|---|---|---|
| | | HL-60 cells per ml × $10^{-4}$ | inhibition of proliferation (%) | formazan "+" cells / total cells counted | % "+" |
| Control (no drug) | | 54.0 ± 1.8 | — | 13/345 | 4 |
| 1α,25S,26—$(OH)_3D_3$ | 1 | 48.0 ± 3.4 | 11 | 19/293 | 7 |
| " | 3 | 50.7 ± 1.9 | 6 | 14/340 | 4 |
| " | 10 | 45.3 ± 1.0 | 16 | 142/349 | 41 |
| 1α,25R,26—$(OH)_3D_3$ | 1 | 50.9 ± 3.8 | 6 | 11/309 | 4 |
| " | 3 | 50.6 ± 1.2 | 7 | 22/315 | 7 |
| " | 10 | 43.2 ± 0.8 | 20 | 138/307 | 45 |
| 1α,25S,26—$(OH)_3$—$\Delta^{22}$—$D_3$ | 1 | 50.7 ± 3.0 | 7 | 12/280 | 4 |
| " | 3 | 49.6 ± 0.8 | 8 | 50/366 | 14 |
| " | 10 | 32.3 ± 0.8 | 40 | 223/347 | 65 |
| None (medium control) | | 86.8 ± 7.2 | — | 3/311 | 1 |
| 1α,25S,26—$(OH)_3$—$D_2$ | 1 | 75.9 ± 1.4 | 12 | 4/302 | 1 |
| " | 10 | 48.0 ± 1.7 | 44 | 97/300 | 32 |
| " | 100 | 22.4 ± 0.1 | 74 | 306/311 | 98 |

a. The vehicle (ethanol) concentration used in this experiment ranged from 0.000001% to 0.01% v/v.

b. The HL—60 cell density at the initiation of these experiments was 1 × $10^4$ per ml except for 1α,25S,26—$(OH)_3$—$D_2$ which had an initial cell density of 2 × $20^4$ cells per ml.

TABLE 2

Anti-proliferative and differentiation-inducing effects of 1α,25S,26—$(OH)_3D_3$; 1α,25R,26—$(OH)_3$—$D_3$ and 1α,25S,26—$(OH)_3$—$\Delta^{22}$—$D_3$ at doses of 10 to 1000 × $10^{-9}$ molar on HL—60 cells, in vitro.

| Compound and[a] concentration (× $10^{-9}$ molar) | | Proliferation[b] | | Differentiation | |
|---|---|---|---|---|---|
| | | HL-60 cells per ml × $10^{-4}$ | inhibition of proliferation (%) | formazan "+" cells / total cells counted | % "+" |
| Control (no drug) | | 32.0 ± 1.3 | — | 3/317 | 1 |
| 1α,25S,26—$(OH)_3D_3$ | 10 | 27.2 ± 1.9 | 15 | 81/383 | 21 |
| " | 30 | 14.5 ± 0.8 | 55 | 365/425 | 86 |
| " | 100 | 10.7 ± 0.8 | 67 | 271/274 | |
| " | 1000 | 8.3± 0.5 | 74 | 342/344 | 99 |
| 1α,25R,26—$(OH)_3D_3$ | 10 | 25.9 ± 1.8 | 19 | 113/336 | 34 |
| " | 30 | 13.6 ± 0.7 | 58 | 296/332 | 90 |
| " | 100 | 9.2 ± 0.3 | 71 | 350/355 | 98 |
| " | 1000 | 7.8 ± 0.2 | 76 | 365/367 | 99 |
| 1α,25S,26—$(OH)_3$—$\Delta^{22}$—$D_3$ | 10 | 19.3 ± 0.5 | 40 | 229/379 | 60 |
| " | 30 | 10.6 ± 0.5 | 67 | 286/297 | 96 |
| " | 100 | 8.8 ± 0.3 | 73 | 378/381 | 99 |
| " | 1000 | 7.2 ± 0.3 | 78 | 299/302 | 99 |

a. The vehicle (ethanol) concentration in this experiment ranged from 0.0001 to 0.01% v/v.
b. The HL—60 cell density at the initiation of this experiment was 1 × $10^4$ per ml.

TABLE 3

Anti-proliferative and differentiation-inducing effects of 1α,25S,26—$(OH)_3D_3$; 1α,25R,26—$(OH)_3D_3$ and 1α,25S,26—$(OH)_3$—$\Delta^{22}D_3$ at doses of 3 to 100 × $10^{-9}$ on HL—60 cells, in vitro.
cells, in vitro.

| Compound and[a] concentration (× $10^{-9}$ molar) | | Proliferation[b] | | Differentiation | |
|---|---|---|---|---|---|
| | | HL-60 cells per ml × $10^{-4}$ (mean ± SD) | inhibition of proliferation (%) | formazan "+" cells total cells counted | % "+" |
| None (vehicle at 0.01%, v/v) | | 26.6 ± 1.0 | — | 4/447 | 1 |
| 1α,25S,26—$(OH)_3D_3$ | 3 | 28.3 ± 0.5 | 0 | 5/449 | 1 |
| " | 10 | 23.7 ± 1.0 | 11 | 69/473 | 15 |
| " | 30 | 20.7 ± 1.0 | 22 | 244/422 | 58 |
| " | 100 | 18.1 ± 0.9 | 32 | 337/415 | 81 |
| 1α,25R,26—$(OH)_3D_3$ | 3 | 26.7 ± 2.0 | 0 | 5/425 | 1 |
| " | 10 | 25.3 ± 0.6 | 5 | 86/454 | 19 |
| " | 30 | 21.1 ± 0.6 | 21 | 314/490 | 64 |
| " | 100 | 16.5 ± 0.4 | 38 | 372/422 | 84 |
| 1α,25S,26—$(OH)_3$—$\Delta^{22}$—$D_3$ | 3 | 24.9 ± 1.2 | 6 | 14/476 | 3 |
| " | 10 | 22.0 ± 0.6 | 17 | 198/607 | 33 |
| " | 30 | 18.5 ± 0.7 | 30 | 319/456 | 70 |
| " | 100 | 16.9 ± 0.7 | 36 | 350/409 | 86 |

a. In this experiment, vehicle (ethanol) concentration was held constant at 0.01% v/v.
b. The HL—60 cell density at the initiation of this experiment was 1 x $10^4$ per ml.

TABLE 4

Anti-proliferative and differentiation-inducing effects of 1α,25S,26—$(OH)_3$—$D_3$; 1α,25R,26—$(OH)_3$—$D_3$; 1α,25S,26—$(OH)_3$—$\Delta^{22}$—$D_3$ and 1α,25S,26—$(OH)_3$—$D_2$ at doses of 1 to 100 × $10^{-9}$ molar on HL—60 cells, in vitro.

| Compound and concentration (× $10^{-9}$ molar) | | Proliferation[c] | | Differentiation | | | |
|---|---|---|---|---|---|---|---|
| | | HL-60 cells per ml × $10^{-4}$ (mean ± SD) | inhibition of proliferation (%) | NBT reduction formazan "+" cells / total cells counted | % "+" | phagocytosis phagocytic cells / total cells counted | % "+" |
| Medium control (no drug) | | 68.5 ± 4.8 | — | 2/277 | <1 | 3/245 | 1 |
| Vehicle control[a] | | 66.3 ± 3.2 | 0 | 2/241 | <1 | 7/240 | 3 |
| 1α,25S,26—$(OH)_3D_3$ | 3 | 68.3 ± 4.0 | 0 | 12/278 | 4 | 13/267 | 5 |
| " | 10 | 70.0 ± 2.2 | 0 | 80/266 | 30 | 78/247 | 32 |
| " | 30 | 43.3 ± 3.9 | 35 | 191/244 | 80 | 196/264 | 74 |
| " | 100 | 29.3 ± 1.7 | 56 | 258/265 | 97 | 220/245 | 90 |
| 1α,25R,26—$(OH)_3D_3$ | 3 | 72.4 ± 0.8 | 0 | 30/342 | 9 | 31/285 | 11 |
| " | 10 | 59.9 ± 3.8 | 10 | 129/245 | 53 | 117/283 | 41 |
| " | 30 | 36.1 ± 1.7 | 46 | 226/258 | 88 | 190/243 | 78 |
| " | 100 | 27.8 ± 0.9 | 58 | 239/247 | 97 | 210/225 | 93 |
| 1α,25S,26—$(OH)_3$—$\Delta^{22}$—$D_3$ | 3 | 59.3 ± 1.6 | 11 | 78/272 | 29 | 71/281 | 25 |
| " | 10 | 50.4 ± 1.1 | 24 | 165/270 | 61 | 152/256 | 59 |
| " | 30 | 35.6 ± 1.6 | 46 | 228/257 | 89 | 214/256 | 84 |
| " | 100 | 28.9 ± 0.6 | 56 | 241/252 | 96 | 247/264 | 94 |
| None (medium control) | | 58.5 ± 1.8 | — | 2/317 | <1 | 2/278 | <1 |
| Vehicle (0.01% ethanol)[b] | | 58.0 ± 2.2 | 0 | 1/309 | <1 | 2/303 | <1 |

TABLE 4 (continued)

Anti-proliferative and differentiation-inducing effects of 1α,25S,26—$(OH)_3$—$D_3$; 1α,25R,26—$(OH)_3$—$D_3$; 1α,25S,26—$(OH)_3$—$\Delta^{22}$—$D_3$ and 1α,25S,26—$(OH)_3$—$D_2$ at doses of 1 to 100 × $10^{-9}$ molar on HL—60 cells, in vitro.

| Compound and concentration (× $10^{-9}$ molar) | | Proliferation[c] | | Differentiation | | | |
|---|---|---|---|---|---|---|---|
| | | HL-60 cells per ml × $10^{-4}$ (mean ± SD) | inhibition of proliferation (%) | NBT reduction formazan "+" cells / total cells counted | % "+" | phagocytosis phagocytic cells / total cells counted | % "+" |
| 1α,25S,26—$(OH)_3$—$D_2$ | 1 | 60.3 ± 1.0 | 0 | 5/320 | 2 | 8/332 | 2 |
| " | 3 | 54.4 ± 1.1 | 7 | 20/310 | 7 | 18/334 | 5 |
| " | 10 | 42.0 ± 0.7 | 28 | 94/310 | 30 | 74/319 | 23 |
| " | 30 | 29.3 ± 1.1 | 50 | 176/308 | 57 | 188/360 | 52 |
| " | 100 | 20.8 ± 0.8 | 64 | 314/322 | 98 | 324/352 | 92 |

a. All cultures (except medium controls) contained 0.001% v/v ethanol as vehicle.
b. All cultures (except medium controls) contained 0.01% v/v ethanol as vehicle.
c. The HL—60 cell density at the initiation of these experiments was 2 × $10^4$ per ml.

The R and S epimers of formula I may be administered to a warm-blooded animal requiring treatment of tumor cells in dosages in the range of 0.05 to 500 micrograms/kg per day. They can be administered orally as well as intramuscularly, intravenously or intraperitoneally, e.g. in form of tablets, capsules, syrups or elixirs for oral administration, or in sterile solutions or suspensions for parenteral administration. About 0.05 to 500 micrograms of R or S epimer of formula I can be compounded with a pharmaceutically acceptable vehicle, carrier, excipient, e.g. dicalcium phosphate; binder, e.g. gum tragacanth; preservative or stabilizer, in a unit dosage. Tablets and capsules can also contain a disintegrating agent, e.g. corn starch; a lubricant, e.g. magnesium stearate; a sweetening agent, e.g. sucrose, and a flavoring agent, e.g. peppermint. Various other materials, such as coatings, e.g. shellac, may be present to modify the physical form of the dosage unit. Sterile compositions for injection can contain water, a naturally-occurring vegetable oil, e.g. sesame oil, or a synthetic fatty vehicle, e.g. ethyl oleate. Buffers, preservatives and antioxidants can also be incorporated.

The Examples which follow further illustrate the invention. All temperatures are in degrees Centigrade.

Example 1

A) To a suspension of 0.122 g of $LiAlH_4$ in 3.2 ml of dry THF at 0° were added 0.336 g of [1R - [1β - (R*,E,S*),3aα,4β,7aβ]] - 6 - [octahydro - 4 - (1,1 - dimethylethyl)dimethylsilyloxy - 7α - methyl - 1H - inden - 1 - yl] - 2 - hydroxy - 2 - methyl - 4 - heptenecarboxylic acid methyl ester and 4 ml of dry THF. After one hour, 0.061 g of $LiAlH_4$ were added, the mixture was stirred for 20 minutes, then the cooling bath was removed. After 2.5 hours, the mixture was cooled to 0° and 0.6 ml of ethyl acetate were added. After stirring, 3.5 ml of saturated ammonium chloride solution were added, the cooling bath was removed and the mixture was stirred, then filtered and washed with chloroform and ethyl acetate. The filtrates were dried, filtered and concentrated. The residue was chromatographed on silica gel with hexane-ethyl acetate, to give [1R - [1β - (R*,E,S*),3aα,4β,7aβ]] - 6 - [octahydro - 4 - [(1,1 - dimethylethyl) - dimethylsilyloxy - 7α - methyl - 1H - inden - 1 - yl] - 2 - methyl - 4 - heptene - 1,2 - diol (89% yield), $[\alpha]_D^{25}$ = +48.75° (c 0.9388, chloroform).

B) To a solution of 0.257 g of the silyl ether obtained in A), 3.6 ml of acetonitrile and 3.0 ml of THF under argon, were added 2.8 ml of 48% aqueous hydrogen fluoride. The mixture was stirred for 3 hours, then poured into 200 ml of chloroform and 20 ml of water. The aqueous phase was extracted with 2 × 100 ml of chloroform and the combined chloroform layers were washed with 20 ml of saturated sodium bicarbonate solution. The extract was dried, filtered and concentrated. The residue was chromatographed with ethyl acetate on silica gel to give 0.176 g (95%) of [1R - [1β - (R*,E,S*),3aα,4β,7aβ]] - 6 - (octahydro - 4 - hydroxy - 7a - methyl - 1H - inden - 1 - yl) - 2 - methyl - 4 - heptene - 1,2 - diol, m.p. 87—88° (methanol-water, 1:1), $[\alpha]_D^{25}$ +28.2° (c 0.748, chloroform).

C) A solution of 0.254 g of the triol obtained in B), 10 ml of 2,2-dimethoxypropane and 16 mg of p-toluenesulfonic acid monohydrate was stirred under argon for 50 minutes. Then 2 ml of methanol were added, the mixture was stirred for 45 minutes, then 2.5 ml of saturated sodium bicarbonate solution were added. The mixture was stirred for 1 hour, then diluted with 150 ml of chloroform and washed with 10 ml of water. The aqueous phase was extracted with 2 × 50 ml of chloroform and the combined chloroform layers were dried. The mixture was filtered, concentrated and chromatographed on silica gel with hexane-ethyl acetate to give 0.259 (90%) of [1R - [1β - (R*,E,S*),3aα,4β,7aβ]] - octahydro - 7a - methyl - 1 - [1 - methyl - 4 - (2,2,4 - trimethyl - 1,3 - dioxolan - 4 - yl) - 2 - butenyl] - 1H - inden - 4 - ol, $[\alpha]_D^{25}$ +21.27° (c 0.6018, chloroform).

D) The butene derivative obtained in C) was hydrogenated at atmospheric pressure over 10% palladium on carbon in ethyl acetate. Filtration and removal of solvent gave quantitatively amorphous [1R - [1β - (R*,S*),3aα,4β,7aβ]] - octahydro - 7a - methyl - 1 - [1 - methyl - 4 - (2,2,4 - trimethyl - 1,3 - dioxolan - 4 - yl) - butyl] - 1H - inden - 4 - ol, $[\alpha]_D^{25}$ +33.3° (c 0.941, chloroform).

E) To a suspension of 1.720 g of 2,2'-bipyridinium chlorochromate and 0.860 g of anhydrous sodium acetate in 10 ml of methylene chloride, was added a solution of 0.500 g of the indenol derivative obtained in D) in 5 ml of methylene chloride and the mixture was stirred for 2 hours. Additional 0.800 g of 2,2'-bipyridinium chlorochromate were added and the stirring continued for 2.5 hours. 1 ml of 2-propanol was added and 15 minutes later, the mixture was diluted with water and extracted with ether. The combined organic phases were dried and evaporated and the residue was purified by filtration through silica with hexane-ethyl acetate to give 0.446 g (90% yield) of pure [1R - (1R*,S*) - 1β,3aα,7aβ] - octahydro - 7a - methyl - 1 - [1 - methyl - 4 - (2,2,4 - trimethyl - 1,3 - dioxolan - 4 - yl) - butyl] - 4H - inden - 4 - one.

F) A solution of 1.430 g of [3S - (3α,5β,Z)] - 2 - [2 - methylene - 3,5 bis - [(1,1 - dimethylethyl)-dimethylsilyloxy]cyclohexylidene]ethyldiphenylphosphine oxide in 30 ml of anhydrous THF was treated dropwise and under argon at −78°C with 1.4 ml of a 1.7 molar solution of n-butyllithium in hexane. Five minutes later, a solution of 0.460 g of the indenone derivative obtained in E) in 5 ml of anhydrous THF were added dropwise and the mixture was stirred at −78° for 2.5 hours, then treated with 5 ml of a 1N aqueous solution of sodium bicarbonate and potassium-sodium tartrate, allowed to come to room temperature and extracted with ethyl acetate. The combined organic extracts were dried, evaporated and the residue was purified by chromatography on silica with hexane-ethyl acetate to give 0.91 g (95%) of pure 1α,25S,26 - trihydroxy - cholecalciferol - 1,3 - dimethyl - t - butylsilyl 25,26 - acetonide.

G) To a solution of 0.91 g of the acetonide obtained in F) in 200 ml of methanol, were added 45 g of a

cation exchange resin and the mixture was stirred under argon for 16 hours. After filtration, the methanol solution was evaporated to dryness and the residue was dissolved in 100 ml of ethyl acetate and washed with brine. The organic phases were combined, dried and evaporated, and the residue was purified by chromatography on silica with ethyl acetate, to give 0.486 g (86% yield) of 1α,25S,26-trihydroxycholecalciferol, m.p. 163—164°, $[\alpha]_D^{25}$ +58.8° (c 0.5, methanol).

Example 2

A) In a manner analogous to that of Example 1E) 0.145 g of the indenol derivative obtained in Example 1C) were converted to 0.134 g (93%) of pure [1R - (1R*,2E,S*)1β,3aα,7aβ] - octahydro - 7α - methyl - 1 - [1 - methyl - 4 - (2,2,4 - trimethyl - 1,3 - dioxolan - 4 - yl) - 2 - butenyl] - 4H - inden - 4 - one, $[\alpha]_D^{25}$ +6,79° (c 0.2, ethanol).

B) In a manner analogous to that of Example 1F) and G), starting from 0.354 g of [3S - (3α,5β,Z)] - 2 - [2 - methylene - 3,5 - bis - [(1,1 - dimethylethyl)dimethylsilyloxy]cyclohexylidene]ethyldiphenylphosphine oxide and from 0.113 g of the indenone derivative obtained in Example 2A), there were obtained 0.131 g (90%) of pure 1α,25S,26 - trihydroxy - $\Delta^{22}$ - cholecalciferol, $[\alpha]_D^{25}$ +44.9 (c 0.2, ethanol).

Example 3

A) A mixture of 7.60 g (30 mmol) of [1R[1α(R*,Z),3aβ,4α,7aα]] - octahydro - 7a - methyl - 1 - [1 - methyl - 3 - (methylimino)propyl] - 1H - inden - 4 - ol - N - oxide, 5.70 g (36 mmol) of methyl mesaconate and 4 ml of xylene was heated at 140°. The obtained solution was heated for 1 hr and then cooled to room temperature. Chromatography on silica gel with $CH_2Cl_2$/EtOAc afforded 5.42 g (44%) of [3S,4S,5S[3β,4α,5β[(2R*),1R*(1β,3aα,4β,7aβ)]]] - 3 - [2 - (octahydro - 4 - hydroxy - 7a - methyl - 1H - inden - 1 - yl) - propyl] - 2,2 - dimethyl - 4,5 - isoxazolidinedicarboxylic acid dimethyl ester, $[\alpha]_D^{25}$ = +126.4° (c 0.911, $CHCl_3$); m/e 411.

B) A solution of 10.53 g (25.6 mmol) of the diester obtained in A) in 30 ml of dry THF was added dropwise over 30 min under argon to a stirred suspension of 4.85 g (129 mmol) of $LiAlH_4$ in 150 ml of dry THF, maintaining the temperature between 3 and 8°. The suspension was stirred for 1 hr and then 6 ml of water followed by 4 ml of 1N NaOH were added. After 15 min, the suspension was filtered. The filter cake was washed with 6 × 25 ml of THF. The combined filtrates, on evaporation of the solvent, afforded 8.32 g of solid residue. The filter cake still contained product and was added to 250 ml of 20% Rochelle salt solution, stirred for 1 hr and then extracted with 3 × 150 ml of ethyl acetate. The combined organic phases were dried, filtered and evaporated to give 0.70 g of residue. Thus, the total of crude [3S,4R,5S[3β,4α,5β][(2R*),(1β,3aα,4β,7aβ)]]] - 3 - [2 - (octahydro - 4 - hydroxy - 7a - methyl - 1H - inden - 1 - yl) - propyl] - 2,2 - dimethyl - 4,5 - isoxazolidinedimethanol amounted to 9.02 g (99%), m.p. 151—152° (EtOAc), $[\alpha]_D^{25}$ = +108.4° (c 0.944, $CHCl_3$), m/e 355.

C) A solution of the dimethanol derivative obtained in B) and 2.5 ml (38 mmol) of methyl iodide in 10 ml of dry THF and 50 ml of toluene, was heated at 60° for 3.5 hr and then was evaporated to dryness. The crude methiodide was dissolved in 250 ml of 50% aqueous acetic acid with warming and stirring. After the solution was cooled to room temperature, 9.0 g (137 mmol) of zinc dust were added. The suspension was stirred overnight and the residual zinc was removed by filtration and washed with aq. HOAc. The pH of the filtrate was adjusted to 11 with 230 ml of conc. ammonium hydroxide. Extraction with 3 × 300 ml of $CH_2Cl_2$, 2 × 300 ml of ether and 2 × 250 ml of $CH_2Cl_2$/i-PrOH gave 8.7 g of crude [1R - [1α(3R*,4S*,5R*,6S*),3aβ,4α,7aα] - 6 - octahydro - 4 - hydroxy - 7a - methyl - 1H - inden - 1 - yl) - 4 - (dimethylamino) - 3 - hydroxymethyl - 2 - methylheptane - 1,2 - diol. Recrystallization from 350 ml of $CH_2Cl_2$ gave 5.90 g of crystals. The mother liquors afforded, after chromatography on silica gel with EtOAc/MeOH/$Et_3N$, 1.62 g for a total of 7.52 g (81% yield) of the product, m.p. 178—180° ($CH_2Cl_2$), $[\alpha]_D^{25}$ = +21.7° (c 0.986, $CHCl_3$).

D) The addition of 3.0 g (15.8 mmol) of p-toluenesulfonic acid monohydrate to a suspension of 4.90 g (13.2 mmol) of the diol obtained in C), in 75 ml of acetone and 4.9 ml of dimethoxypropane, produced a solution which was stirred under argon for 17 hrs. Then, 8 ml of 2N NaOH were added and the mixture was concentrated. The residual aqueous suspension was extracted with 4 × 100 ml of methylene chloride. The combined organic phases were dried, filtered and evaporated to give 5.7 g of product. Purification by medium pressure LC on silica gel with hexane/EtOAc/$Et_3N$, gave 4.79 g (88%) of [1R - [1α(1R*,3S*,4R*)[4S*],3aβ,4α,7aα]] - octahydro - 7a - methyl - 1 - [3 - (dimethylamino) - 4 - hydroxymethyl - 1 - methyl - 4 - (2,2,4 - trimethyl - 1,3 - dioxolan - 4 - yl) - butyl] - 1H - inden - 4 - ol, m.p. 104—105° (pentane/ether, 2:1), $[\alpha]_D^{25}$ = +19.5° (c 0.943, $CHCl_3$).

E) A solution of the butylindenol derivative obtained in D) and 0.5 ml (7.5 mmol) of methyl iodide in 20 ml of dry toluene and 50 mg of anhydrous potassium carbonate was heated at 65° for 16 hr under argon. An additional 0.5 ml (7.5 mmol) of methyl iodide was added and heating was continued for 4.5 hr. The reaction mixture was evaporated. To the obtained residue were added 10 ml of t-butanol and 1.12 g (10 mmol) of potassium t-butylate. The reaction mixture was heated under argon at 55° for 24 hrs, then at reflux for 5 hrs. The t-butanol was evaporated. To the residue were added 10 ml of water. It was extracted with 3 × 50 ml of ether. The combined extracts were dried, filtered and evaporated to give 1.63 g of crude product. Medium pressure LC on silica gel with $CH_2Cl_2$/EtOAc gave 881 mg (48%) of [1R - [1α(1R*,E,4R*),[4S*],3aβ,4α,7aα]] - octahydro - 7a - methyl - 1 - [4 - (hydroxymethyl) - 1 - methyl - 4 -

[(2,2,4 - trimethyl - 1,3 - dioxolan - 4 - yl) - 2 - butenyl] - 1H - inden - 4 - ol.

F) To a stirred solution of 742 mg (2.0 mmol) of the indenol derivative obtained in E) and 613 mg (6.0 mmol) of triethylamine in 10 ml of methylene chloride, was added at 0° under argon, a solution of 385 mg (2.0 mmol) of p-toluenesulfonyl chloride (TsCl) in 4 ml of methylene chloride. After 45 min at 0°, the bath was removed. After 5 hr at room temperature, 204 mg (2 mmol) of triethylamine and 385 mg (2 mmol) of TsCl were added. After 22 hr, 204 mg of triethylamine and 385 mg of TsCl were added. After 7 hrs, the reaction mixture was poured into 10 ml of 1N NaOH. After separation of the phases, the aqueous phase was extracted with 2 × 20 ml of methylene chloride. The extracts were washed with 10 ml of 1N NaOH. The combined organic extracts were dried, filtered and evaporated to give 1.35 g of crude product. LC separation on silica gel with hexane/EtOAc, gave 840 mg (80%) of [1R - [1α(1R*,E,4R*),[4S*],3aβ,4α,7aα]] - octahydro - 7a - methyl - 1 - [1 - methyl - 4 - [[[4 - methylphenyl)-sulfonyl]oxy]methyl] - 4 - [(2,2,4-trimethyl - 1,3 - dioxolan - 4 - yl) - 2 - butenyl] - 1H - inden - 4 - ol.

G) To a stirred suspension of 240 mg (6.3 mmol) of $LiAlH_4$ in 15 ml of THF under argon, was added a solution of 818 mg (1.57 mmol) of the tosylate obtained in F). The suspension was heated at reflux for 3 hrs and then cooled to 0°. Then, 0.4 ml of water followed by 0.6 ml of 1N NaOH were added. The resulting mixture was stirred for 15 min and then filtered. The filter cake was washed with 2 × 50 ml of THF. The combined filtrates, on evaporation, gave 641 mg of crude product. Chromatography on silica gel with hexane/EtOAc, afforded 498 mg (90%) of [1R - [1α(1R*,E,4S*),[4S*],3aβ,4α,7aα]] - octahydro - 7a - methyl - 1 - [1,4 - dimethyl - 4 - [(2,2,4 - trimethyl - 1,3 - dioxolan - 4 - yl) - 2 - butenyl] - 1H - inden - 4 - ol, m.p. 69—71° (hexane), $[\alpha]_D^{25} = +5.18$ (c 1.023, $CHCl_3$).

H) In a manner analogous to that of Example 1E), from 1.5 g (5.13 mmol) of 2,2′-dipyridinium chlorochromate and 0.300 g (0.86 mmol) of the indenol derivative obtained in Example 3G), there were obtained 0.284 g (95%) of [1R - [1β(1R*,2E,4S*),[4S*],3aα,7aβ]] - octahydro - 7a - methyl - 1 - [1,4 - dimethyl - 4 - [(2,2,4 - trimethyl - 1,3 - dioxolan - 4 - yl) - 2 - butenyl] - 4H - inden - 4 - one, $[\alpha]_D^{25} = -12.1°$ (c 0.5 in ethanol).

I) In a manner analogous to that of Example 1F) and G), from 665 mg (1.14 mmol) of [3S - (3α,5β,Z)] - 2 - [2 - methylene - 3,5 - bis[(1,1 - dimethylethyl)dimethylsilyloxy]cyclohexylidene] - ethyldiphenyl phosphine oxide and 234 mg (0.671 mmol) of the indenone derivative obtained in Example 3H), there were obtained 248 mg (83%) of pure 1α,25S,26-trihydroxyergocalciferol, m.p. 186—187°; $[\alpha]_D^{25} = +40.2$ (c 0.3 in ethanol); $^1H$ NMR (200 MHz, $CD_3OD$) δ 0.58 (s, 3H), 0.98 (d, J=7.2Hz, 3H) 1.06 (d, J=7.6, 3H), 1.08 (s, 3H), 3.40 (AB q, $J_{AB}$=8.4Hz, $\Delta_\gamma$=16.0Hz, 2H), 4.14 (m, 1H), 4.34 (m, 1H), 4.90 (s, 1H), 5.26 (dd, $J_1$=8.4Hz, $J_2$=15.6Hz, 1H), 5.29 (s, 1H), 5.45 (dd, $J_1$=7.9Hz, $J_2$=15.6Hz, 1H), 6.08 (d, J=11.6Hz, 1H), 6.32 (d, J=11.6Hz, 1H).

## Example A
### Injectable Dosage Form Formulation

| Ingredient: | Amount/ml: |
|---|---|
| Sodium chloride | 1.5 mg |
| Monobasic sodium phosphate | 9.2 mg |
| Disodium salt of EDTA | 1.0 mg |
| Isoascorbic Acid | 10.0 mg |
| Tween 20 (polyalkylene derivative of hexitol anhydride long chain fatty acid esters) | 4.0 mg |
| 1α,25S,26-$(OH)_3$-$\Delta^{22}$-$D_3$,1α,25S,26-$(OH)_3$-$D_3$ or 1α,25S,26-$(OH)_3$-$D_3$ | at desired concentration |
| Sodium Hydroxide | q.s. pH 7.0 |
| Water for Injection | q.s. ad |

Example B
Oral Dosage Form Formulation

| | mg/capsule: |
|---|---|
| 1α,25R,26-$(OH)_3$-$\Delta^{22}$-$D_3$,1α,25S,26-$(OH)_3$-$D_3$ or 1α,25R,26-$(OH)_3D_3$ | at desired concentration |
| Neobee M5 (medium chain tri-glycerides) | 200.00 |
| Butylated hydroxyanisole | 0.01 |
| Butylated hydroxytoluene | 0.01 |

**Claims**

1. A process for the preparation of calciferol derivatives of the formula

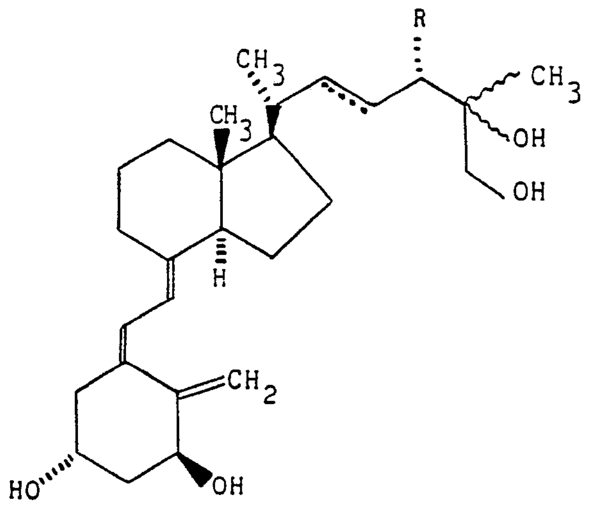

I

wherein R is hydrogen and the dotted line in the side chain is an optional additional C—C-bond, or R is methyl and the dotted line in the side chain is an additional C—C-bond, in the 25R or 25S epimeric form or as mixtures thereof, which comprises reacting the corresponding specific epimer of the formula

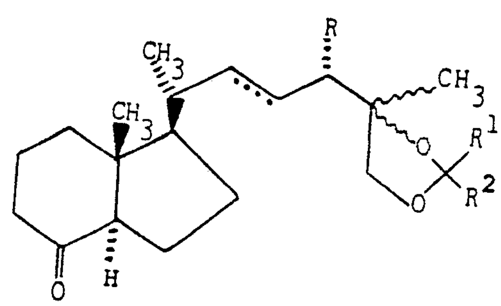

II

wherein R and the dotted line in the side chain have the above significance and $R^1$ and $R^2$ are hydrogen, lower alkyl or aryl, or taken together are $C_{3-6}$-alkylene, with a compound of the formula

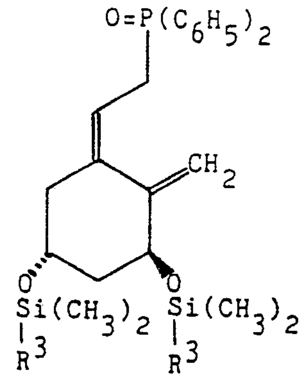

III

wherein $R^3$ is lower alkyl or aryl, and treating the obtained compound of the formula

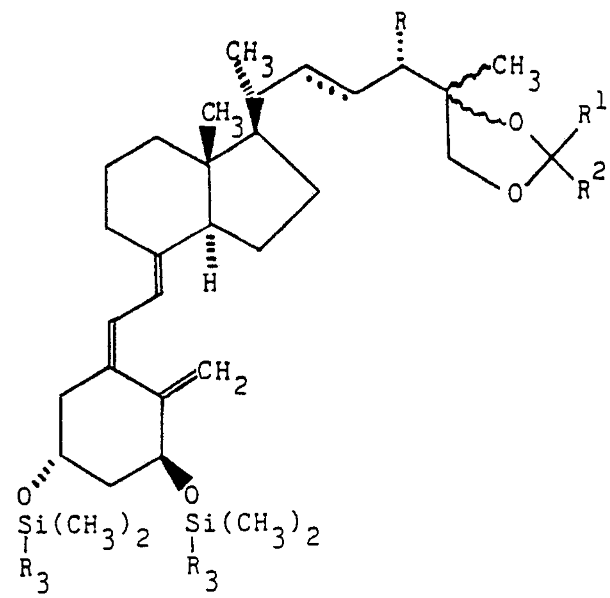

IV

wherein R, $R^1$, $R^2$, $R^3$ and the dotted line in the side chain have the above significance, with a deprotecting agent.

2. A process as in claim 1, wherein R is hydrogen.
3. A process as in claim 2, wherein there is no additional C—C-bond in the side chain.
4. A process as in claim 1, 2 or 3, wherein a 25(S)-epimer of formula I is prepared.
5. Calciferol derivatives of the formula

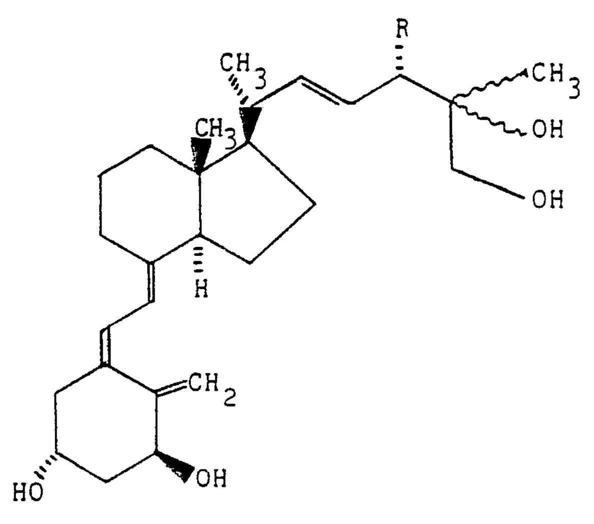

I-A

wherein R is hydrogen or methyl, in the 25R or 25S epimeric form or as mixtures thereof.

6. Calciferol derivatives as in claim 5, wherein R is hydrogen.

7. A compound as in claim 6, 1α,25(S),26-trihydroxy-$\Delta^{22}$-cholecalciferol.

8. A compound as in claim 5, 1α,25(S),26-trihydroxyergocalciferol.

9. Ketones of the formula

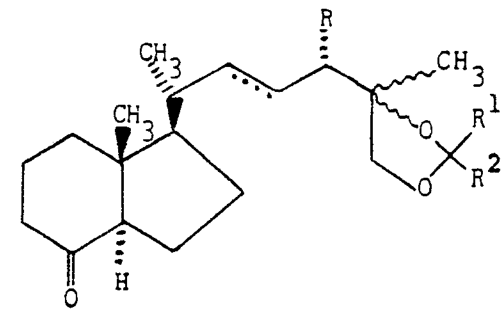

II

wherein R is hydrogen and the dotted line in the side chain is an optional additional C—C-bond, or R is methyl and the dotted line in the side chain is an additional C—C-bond, in the R or S epimeric form or as mixtures thereof.

10. Ketones as in claim 9 wherein R is hydrogen, particularly [1R - (1R*,S*) - 1β,3aα,7aβ] - octahydro - 7a - methyl - 1 - [1 - methyl - 4 - (2,2,4 - trimethyl - 1,3 - dioxolan - 4 - yl) - butyl] - 4H - inden - 4 - one and [1R - (1R*,2E,S*),1β,3aα,7aβ] - octahydro - 7a - methyl - 1 - [1 - methyl - 4 - (2,2,4 - trimethyl - 1,3 - dioxolan - 4 - yl) - 2 - butenyl] - 4H - inden - 4 - one.

11. A compound as in claim 9, [1R - [1β(1R*,2E,4S*),[4S*],3aα,7aβ]] - octahydro - 7a - methyl - 1 - [1,4 - dimethyl - 4 - (2,2,4 - trimethyl - 1,3 - dioxolan - 4 - yl) - 2 - butenyl] - 4H - inden - 4 - one.

12. A compound of formula IA as in any one of claims 5—8, as a pharmaceutically active substance.

13. A compound of formula IA as in any one of claims 5—8, as an anti-proliferative and differentiation-inducing active substance.

14. 1α,25(S),26- or 1α,25(R),26-Trihydroxycholecalciferol, as an anti-proliferative and differentiation-inducing active substance.

15. A medicament containing a compound of formula IA as in any one of claims 5—8.

16. A medicament as in claim 15 for the treatment of tumors.

17. A medicament for treating tumors containing 1α,25(S),26- or 1α,25(R),26-trihydroxycholecalciferol.

18. Use of 1α,25(S),26- or 1α,25(R),26-trihydroxycholecalciferol for the manufacture of an anti-proliferative and differentiation-inducing active medicament.

**Patentansprüche**

1. Ein Verfahren zur Herstellung von Calciferolderivaten der Formel

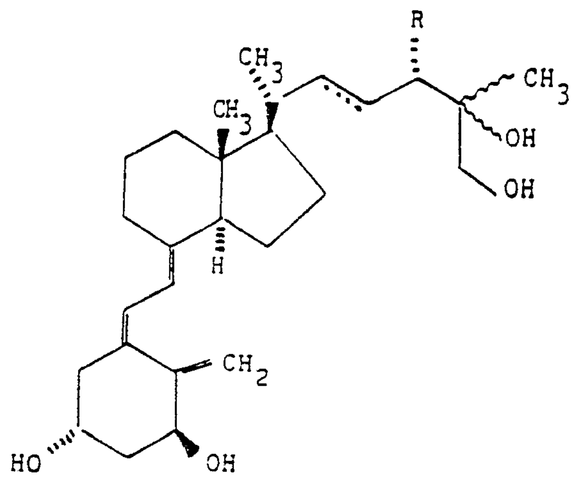

I

III

worin R Wasserstoff und die punktierte Linie in der Seitenkette eine fakultative zusätzliche C—C-Bindung ist, oder R Methyl und die punktierte Linie in der Seitenkette eine zusätzliche C—C-Bindung ist, in der 25 R oder 25 S epimeren Form oder als Gemische davon, das darin besteht, dass man das entsprechende spezifische Epimere der Formel

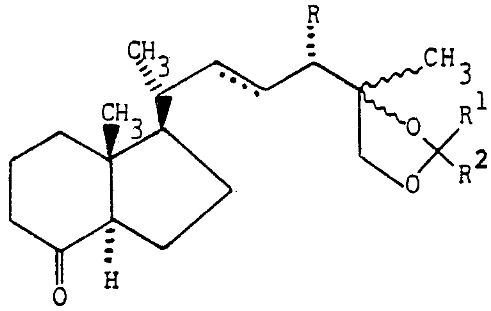

II

worin R und die punktierte Linie in der Seitenkette die obige Bedeutung haben und $R^1$ und $R^2$ Wasserstoff, nieder-Alkyl oder Aryl, oder zusammen $C_{3-6}$-Alkylen sind, umsetzt mit einer Verbindung der Formel

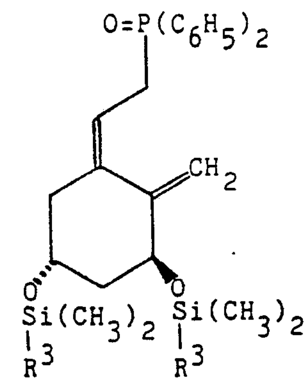

III

worin $R^3$ nieder-Alkyl oder Aryl ist, und die erhaltene Verbindung der Formel

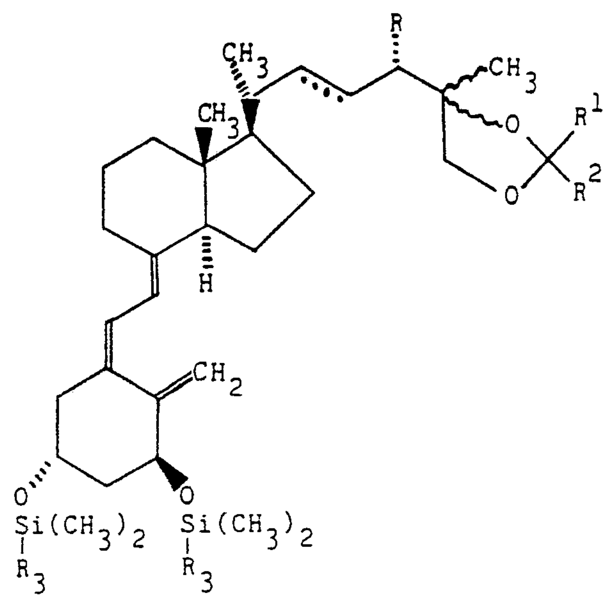

IV

worin R, $R^1$, $R^2$, $R^3$ und die punktierte Linie in der Seitenkette die obige Bedeutung haben, mit einem abspaltenden Mittel behandelt.

2. Ein Verfahren, wie im Anspruch 1, worin R Wasserstoff ist.
3. Ein Verfahren, wie im Anspruch 2, worin die Seitenkette keine zusätzliche C—C-Bindung enthält.
4. Ein Verfahren, wie in Anspruch 1, 2 oder 3, worin man ein 25(S)-Epimeres der Formel I herstellt.

5. Calciferolderivate der Formel

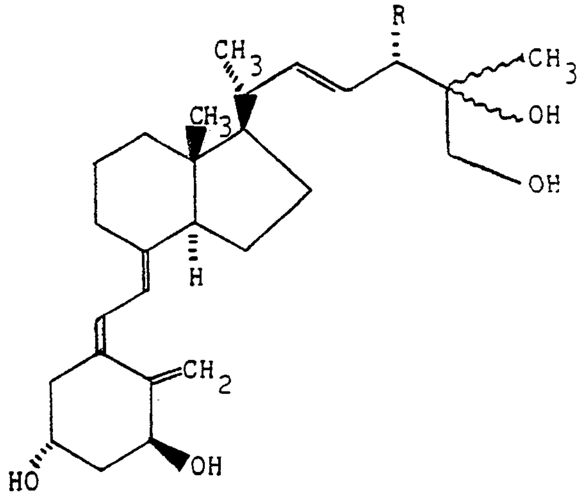

I-A

worin R Wasserstoff oder Methyl ist,
in der 25 R oder 25 S epimeren Form oder als Gemische davon.

6. Calciferolderivate wie in Anspruch 5, worin R Wasserstoff ist.

7. Eine Verbindung wie in Anspruch 6, 1α,25(S),26-Trihydroxy-$\Delta^{22}$-cholecalciferol.

8. Eine Verbindung wie in Anspruch 5, 1α,25(S),26-Trihydroxyergocalciferol.

9. Ketone der Formel

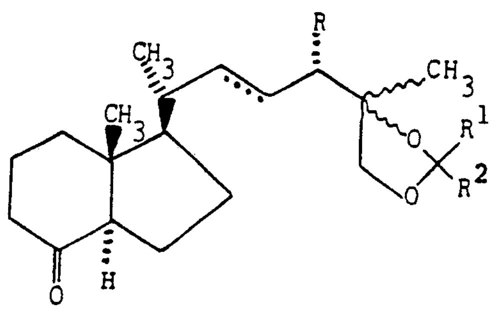

II

worin R Wasserstoff und die punktierte Linie in der Seitenkette eine fakultative zusätzliche C—C-Bindung ist, oder R Methyl und die punktierte Linie in der Seitenkette eine zusätzliche C—C-Bindung ist, in der R oder S epimeren Form oder als Gemische davon.

10. Ketone wie in Anspruch 9, worin R Wasserstoff ist, insbesondere [1R - (1R*,S*) - 1β,3aα,7aβ] - Octahydro - 7a - methyl - 1 - [1 - methyl - 4 - (2,2,4 - trimethyl - 1,3 - dioxolan - 4 - yl) - butyl] - 4H - inden - 4 - on und [1R - (1R*,2E,S*),1β,3aα,7aβ] - Octahydro - 7a - methyl - 1 - [1 - methyl - 4 - (2,2,4 - trimethyl - 1,3 - dioxolan - 4 - yl) - 2 - butenyl] - 4H - inden - 4 - on.

11. Eine Verbindung wie in Anspruch 9, [1R - [1β(1R*,2E,4S*), - [4S*],3aα,7aβ]] - Octahydro - 7a - methyl - 1 - [1,4 - dimethyl - 4 - (2,2,4 - trimethyl - 1,3 - dioxolan - 4 - yl) - 2 - butenyl] - 4H - inden - 4 - on.

12. Eine Verbindung der Formel IA wie in einem der Ansprüche 5—8, als pharmazeutisch aktive Substanz.

13. Eine Verbindung der Formel IA wie in einem der Ansprüche 5—8, als antiproliferativ und differenzierung — induzierend aktive Substanz.

14. 1α,25(S),26- oder 1α,25(R),26-Trihydroxycholecalciferol als antiproliferativ und differenzierung — induzierend aktive Substanz.

15. Ein Medikament enthaltend eine Verbindung der Formel IA wie in einem der Ansprüche 5—8.

16. Ein Medikament wie in Anspruch 15 zur Behandlung von Tumoren.

17. Ein Medikament zur Behandlung von Tumoren enthaltend 1α,25(S),26- oder 1α,25(R),26-Trihydroxycholecalciferol.

18. Verwendung von 1α,25(S),26- oder 1α,25(R),26-Trihydroxycholecalciferol für die Herstellung eines antiproliferativ und differenzierung-induzierenden aktiven Medikaments.

**Revendications**

1. Procédé pour la préparation de dérivés du calciférol de formule

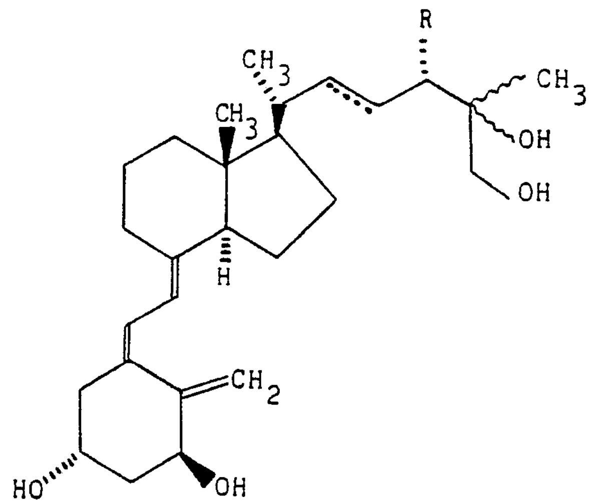

I

dans laquelle R est un atome d'hydrogène et le trait en pointillé dans la chaîne latérale est une liaison C—C facultative supplémentaire, ou R est le groupe méthyle et le trait en pointillé dans la chaîne latérale est une liaison C—C supplémentaire,
sous la forme épimère 25R ou 25S ou sous la forme de leurs mélanges, qui comprend la réaction de l'épimère particulier correspondant de formule

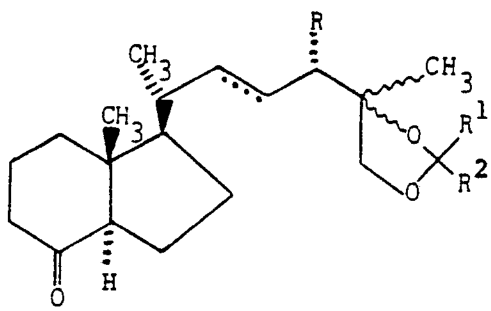

II

dans laquelle R et le trait en pointillé dans la chaîne latérale ont la signification donnée ci-dessus et $R^1$ et $R^2$ sont un atome d'hydrogène, un groupe alkyle inférieur ou aryle, ou pris conjointement sont un groupe alkylène en $C_{3-6}$,
avec un composé de formule

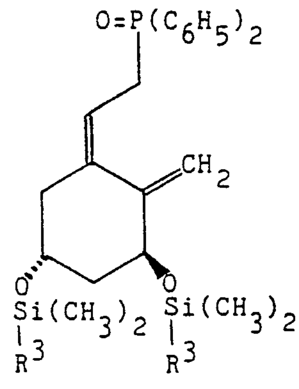

III

dans laquelle $R^3$ est un groupe alkyle inférieur ou aryle,
et le traitement du composé obtenu de formule

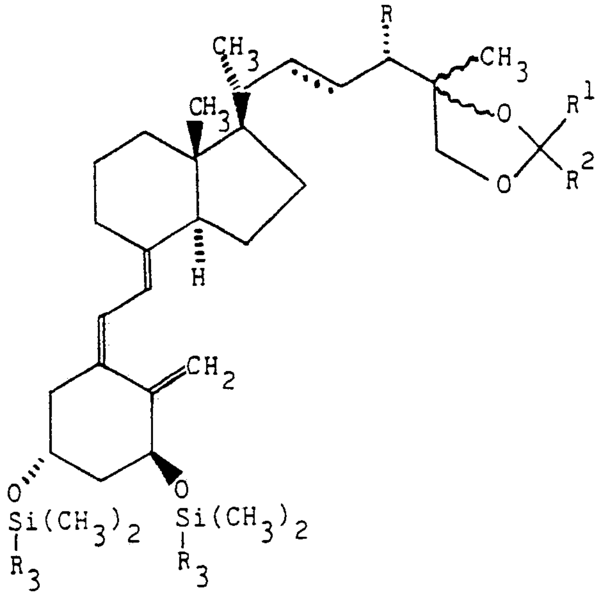

IV

dans laquelle R, $R^1$, $R^2$, $R^3$ et le trait en pointillé, dans la chaîne latérale ont les significations données ci-dessus,
avec un agent de déprotection.

2. Procédé selon la revendication 1, dans lequel R est un atome d'hydrogène.

3. Procédé selon la revendication 2, dans lequel il n'y a pas de liaison C—C supplémentaire dans la chaîne latérale.

4. Procédé selon l'une quelconque des revendications 1, 2 ou 3, dans lequel on prépare un épimère 25(S) de formule I.

5. Dérivés du calciférol de formule

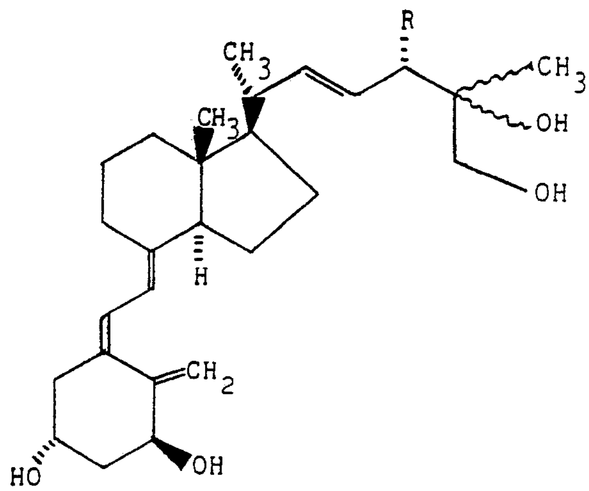

I-A

dans laquelle R est un atome d'hydrogène ou le groupe méthyle,
sous la forme épimère 25R ou 25S ou sous la forme de leurs mélanges.

6. Dérivés du calciférol selon la revendication 5, dans lesquels R est un atome d'hydrogène.

7. Composé selon la revendication 6, qui est le 1α,25(S), 26-trihydroxy-$\Delta^{22}$-cholécalciférol.

8. Composé selon la revendication 5, qui est le 1α,25(S),26-trihydroxy-ergocalciférol.

9. Cétones de formule

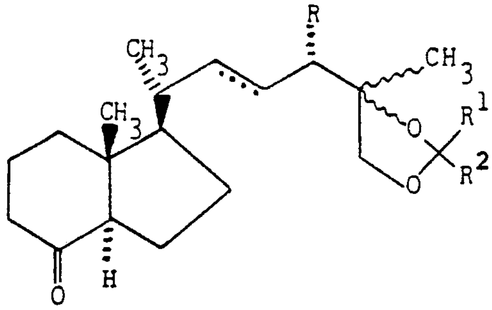

II

dans laquelle R est un atome d'hydrogène et le trait en pointillé, dans la chaîne latérale est une liaison C—C supplémentaire facultative ou R est le groupe méthyle et le trait en pointillé dans la chaîne latérale est une liaison C—C supplémentaire,
sous la forme épimère R ou S ou sous la forme de leurs mélanges.

10. Cétones selon la revendication 9, dans lesquelles R est un atome d'hydrogène, en particulier la (1R - (1R*,S*) - 1β,3aα,7aβ) - octahydro - 7a - méthyl - 1 - (1 - méthyl - 4 - (2,2,4 - triméthyl - 1,3 - dioxolan - 4 - yl) - butyl) - 4H - inden - 4 - one et
la (1R - (1R*,2E,S*),1β,3aα,7aβ) - octahydro - 7a - méthyl - 1 - (1 - méthyl - 4 - (2,2,4 - triméthyl - 1,3 - dioxolan - 4 - yl) - 2 - buténÿl) - 4H - inden - 4 - one.

11. Composé selon la revendication 9, qui est la (1R - (1β(1R*,2E,4S*), - (4S*),3aα,7aβ)) - octahydro - 7a - méthyl - 1 - (1,4 - diméthyl - 4 - (2,2,4 - triméthyl - 1,3 - dioxolan - 4 - yl) - 2 - buténÿl) - 4H - inden - 4 - one.

12. Composé de formule IA selon l'une quelconque des revendications 5 à 8, à titre de substance pharmaceutiquement active.

13. Composé de formule IA selon l'une quelconque des revendications 5 à 8, à titre de substance active comme anti-prolifératif et inducteur de différenciation.

14. 1α,25(S),26- ou 1α,25(R),26-trihydroxycholécalciférol, à titre de substance active comme anti-prolifératif et inducteur de différenciation.

15. Médicament contenant un composé de formule 1A selon l'une quelconque des revendications 5 à 8.

16. Médicament selon la revendication 15, pour le traitement des tumeurs.

17. Médicament pour le traitement des tumeurs contenant de 1α,25(S),26- ou 1α,25(R),26-trihydroxy-cholécalciférol.

18. Utilisation du 1α,25(S),26- ou 1α,25(R),26-trihydroxycholécalciférol, pour la fabrication d'un médicament actif comme anti-prolifératif et inducteur de différenciation.